# EUROPEAN PATENT APPLICATION

(11) **EP 1 892 231 A1**
(43) Date of publication of application: **27.02.2008**
(21) Application number: 06076607.8
(22) Date of filing: 21.08.2006
(51) Int. Cl.: C07C 17/18, B01J 14/00

(54) **A large scale method for the deoxofluorination of ketones**

(71) Applicant: Bayer Schering Pharma Aktiengesellschaft, 13353 Berlin (DE)
(72) Inventor: Lovis, Kai, 12169 Berlin (DE); Geisler, Jens, 13587 Berlin (DE); Senzel, Ralf, 13158 Berlin (DE); Formell, Michael, 13353 Berlin (DE); Finke, Christian, 16567 Schönfliess (DE); Nau, Michael, 13187 Berlin (DE); Schulz, Kathrin, 10439 Berlin (DE); Budde, Uwe, 16348 Wandlitz (DE); Thiel, Uwe, 10245 Berlin (DE)

(57) **Abstract**

A method for the deoxofluorination of a ketone for the production of a geminal difluoride compound in an industrial scale comprising the following steps:
- preparing an educt mixture comprising a ketone and a dialkylaminosulfur trifluoride,
- letting flow the educt mixture into a reaction chamber of defined length, diameter and volume, with a flow path having a width perpendicular to the direction of the flow in the range of 1-10 mm, at a temperature of at least 75 °C up to 120 °C, preferably between 80 and 100 °C, to perform the deoxofluorination reaction and obtaining a product mixture
- to quench the product mixture with a basic aqueous solution in a cooled reservoir vessel.

## Description

### Technical field

The present invention refers to a large scale method for making compounds having geminal fluorine atoms, more particularly it refers to a method comprising the use of diethylaminosulfur trifluoride (DAST) and analogues thereof for the deoxofluorination of ketones to obtain geminal difluoride compounds, said method being performed in an industrial scale.

The present invention further refers to a reaction system for the production in an industrial scale of a geminal difluoride compound and to the use of said reaction system for the deoxofluorination of a ketone by means of a dialkylaminosulfur trifluoride.

### Background art

Fluorination is a common reaction step in which one or more fluorine atoms or fluorinated groups are added to a compound using reagents which act as donors of such fluorine atoms or fluorinated groups.

Several fluorinating reagents in the form of a liquid or of a gas are known in the art, for example fluorine in its elementary form, hydrogen fluoride acid, SF₄, dialkylaminosulfur trifluorides and analogues thereof. The most common reaction is monofluorination, not so common is a geminal difluorination and even more unusual a trifluorination.

The geminal difluoride group can confer to the desired product special properties over those usually offered by a monofluorinated molecule. For example a geminal difluoride group can be advantageously used for further reactions like for example a dehydrofluorination in a intermediate step to the final product.

Geminal difluorination is usually achieved by means of a deoxoflurination reaction that when performed on a aldehyde group result in a terminal difluoride group; starting from a ketone a difluoride group will be obtained.

A class of reagent which is commonly used to perform deoxofluorination reactions is the one of the dialkylaminosulfur trifluorides. The most common reagent of this class is diethylaminosulfur trifluoride (Et-DAST or simply DAST).

The DAST and DAST analogues have proven to be useful reagents for effecting deoxofluorinations; DAST and DAST analogues are liquids which can be used at atmospheric pressure and at near ambient to relatively low temperature (room temperature or below) for most applications.

Geminal difluorinations with DAST and DAST analogues are normally carried out at room temperature or higher. Numerous structurally diverse aldehydes and ketones have been successfully fluorinated with DAST. These include acyclic, cyclic, and aromatic compounds. Elimination does occur to a certain extent when aldehydes and ketones are fluorinated and olefinic byproducts are also observed in these instances.

However, several well-recognized restrictions associated with the use of DAST and DAST analogues limit their versatility in deoxofluorination reactions, particularly in the deoxofluorination of ketones which usually requires drastic conditions with high temperature, excess of the fluorinating agent and longer reaction time.

Since DAST compounds can decompose violently, they are known in the art to be unpractical for large scale industrial use in view of security reason.

The instability of DAST and DAST analogues is reported by MESSINA, P.A., et al. Aminosulfur trifluorides: relative thermal stability. Journal of Fluorine Chemistry. 1989, vol.42, p.137-143. Said decomposition occurs in two steps. A first non-energetic step in which an instable difluoride derivative is formed and a second step where said difluoride undergoes a vigorous exothermic decomposition (detonation).

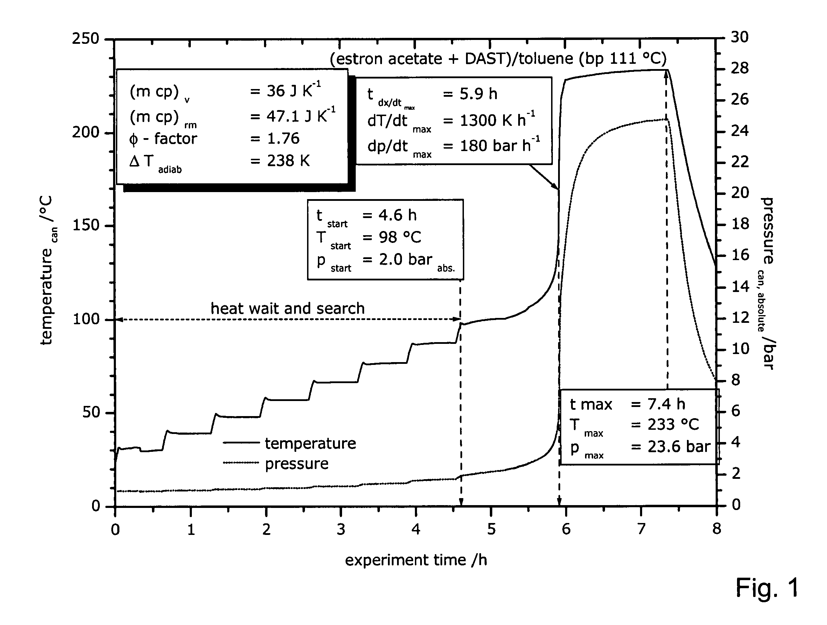

Similar results were obtained in a test performed in our laboratories were the temperature and pressure profile of a mixture of estrone acetate and DAST in toluene was investigated (Fig. 1). The results show that the thermal potential of decomposition of DAST is extremely high and a fatal "runaway" of the reaction is possible suggesting the use of DAST only in lab scale.

EP 1172369 A describes a process for deoxofluorinating a C2-hydroxyl group of a furanose, including: (a) mixing the furanose and a deoxofluorinating agent in a solvent to form a reaction mixture, and (b) heating the reaction mixture to greater than about 50°C, particularly at 90°C for two hours.

However EP 1172369 A describes only the deoxofluorination of a hydroxyl group which requires a very limited amount of DAST. On the contrary, the deoxofluorination of a ketone requires a molar ratio of DAST which is usually more than the double in respect to the ketone.

A method for fluorination reactions is described in EP 1028801 A (BRITISH NUCLEAR FUELS PLC (GB)). Said method comprises carrying out a chemical reaction between at least two fluids, by providing respective flow paths for the at least two fluids, said flow paths communicating with each other in a region in which the at least two fluids may contact each other, and flowing the at least two fluids along said flow paths such that in said region the at least two fluids contact each other and a chemical reaction occurs between them, said region having a width perpendicular to the direction of flow in the range 10-10,000 micrometers. According to EP 1028801 A , using a so-called "microreactor", that is a reactor having dimensions perpendicular to the flow direction of less than 10,000 micrometers, an improved control over a fluid chemical reaction and better product's yield and/or purity can be obtained.

None of the previously cited documents describe an effective and safe method for the deoxofluorination of ketones for the production of geminal difluorides in an industrial scale and there is a clear need in the chemical industrial field for such a method.

A method or a reaction system intended for industrial scale or large scale is a method or reaction system suited for the production of batches of active compound to be used for the manufacture of the final medicinal product.

### Disclosure of the invention

The object of the present invention is a method for the deoxofluorination of ketones for the production of geminal difluoride in an industrial scale according to the independent claim 1.

Claims 2 to 12 refer to particularly favourable forms of embodiment of the method according to the invention.

A further object of the present invention is a reaction system for the production of a geminal difluoride compound in an industrial scale according to independent claim 13.

Claims 13 to 19 refer to advantageous forms of embodiment according to the reaction system of claim 13.

A third object of the present invention is the use of a reaction system according to claims 13-19 for the deoxofluorination of a ketone for the production in an industrial scale of a geminal difluoride product by means of a dialkylaminosulfur trifluoride according to independent claim 20.

The method and the reaction system according to the present invention allow to perform a deoxofluorination reaction in an industrial scale which under the usual methods and known reaction devices would not be possible due to safety reasons.

Furthermore, the present method and reaction system can be used in all the fluorination reaction with DAST or DAST-analogues which have to be performed above the handling temperature of DAST or DAST analogues.

The handling temperature is considered as the temperature at which the use of the reagent can be performed without decomposition and quality alterations and is usually considered around 80 °C under the decomposition temperature of said reagent.

The method and the reaction system according to the invention are used in the chemical and pharmaceutical industry for the production of compounds or intermediates in an industrial scale.

According to the present invention, fluorination and deoxofluorination reaction with DAST or DAST-Analogues which have to be carried out above the handling temperature can be performed in an industrial scale.

The method according to the invention comprises the following steps:
- preparing an educt mixture comprising a ketone and a dialkylaminosulfur trifluoride,
- letting flow the educt mixture into a reaction chamber of defined length, diameter and volume, with a flow path having a width perpendicular to the direction of the flow in the range of 1-10 mm, at a temperature of at least 75 °C up to 120°C, preferably between 80 and 100 °C, to perform the deoxofluorination reaction and obtaining a product mixture
- quenching the product mixture with a basic aqueous solution for example a solution of NaOH 20% w/w in a cooled reservoir vessel.

According to a particularly favourable form of embodiment of the invention, a ketone and a dialkylaminosulfur trifluoride are mixed continuously directly before the reaction chamber to form the educt mixture.

The dialkylaminosulfur trifluoride used in the educt mixture is preferably diethylaminosulfur trifluoride or bis(2-methoxyethyl)-aminosulfur trifluoride.

The ketone is preferably estrone or an ester thereof, particularly estrone acetate.

The educt mixture can further comprise an organic solvent, preferably toluene.

Before entering in the reaction chamber, the educt mixture is advantageously filtered, this avoids the blockage of the reaction system due to precipitates or impurities in the mixture.

Furthermore, the educt mixture and the reaction system in use, are kept under pressure between 0.05 to 10 bar above ambient pressure, preferably under a pressure between 0.1 to 0.5 bar above ambient pressure to assure a constant flow in the system, this can be achieved by using a pressure valve after the reaction chamber for example or by means of a pump placed before the reaction chamber.

The length of the reaction chamber, according to a favourable embodiment of the invention, is comprised between 1 and 30 meters and the diameter between 1 to 10 mm. The total volume of the reaction chamber is advantageously between 0.1 to 10 litres, preferably between 0.2 to 2 litres. The reaction chamber is for example a spirally wound stainless steel tube.

In a reaction system according to the invention, the flow rate of the educt mixture within the reaction chamber can favourably be between 0.1 and 10 litres per hour, preferably between 0.2 to 1 litre per hour, while the residence time of the educt mixture in the reaction chamber can be between 0.1 and 10 hours, preferably between 0.5 and 2 hours.

The reaction system in use is kept advantageously under inert condition by means of an inert gas, for example under nitrogen

### Brief description of the drawings

Other features and advantages of the invention will become evident on reading the following description of one embodiment of the invention, given as a non-binding example, with the help of the enclosed drawings, in which:
- figure 1 shows the results of a test performed to investigate the temperature and pressure profile of a mixture of estron acetate and DAST in toluene;
- figure 2 shows a schematic view of three reaction systems set in parallel according to the invention.

### Mode(s) for carrying out the invention

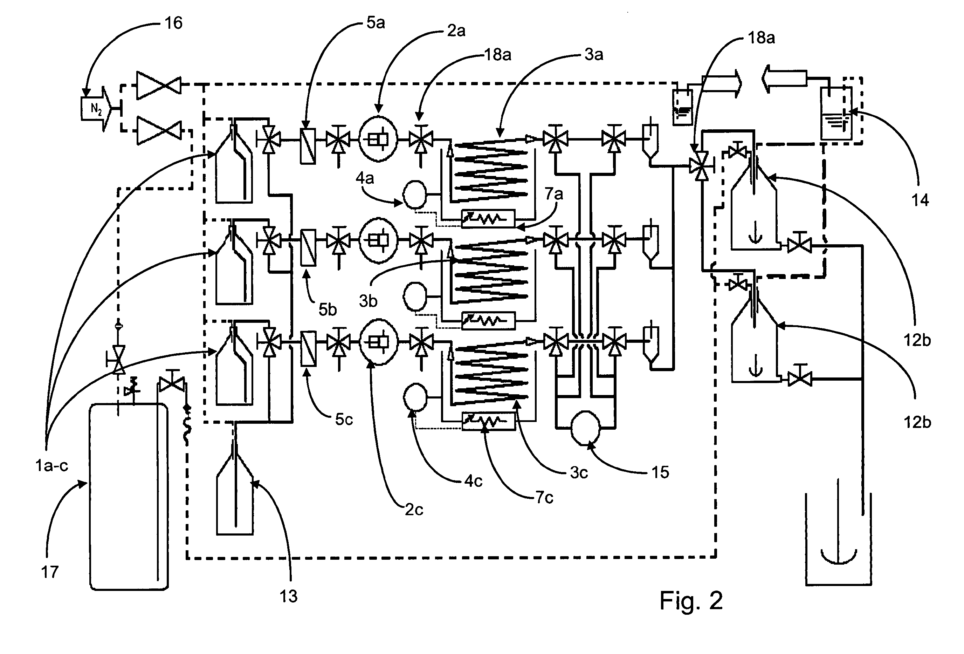

The deoxofluorination of ketones for the production of geminal difluoride in an industrial scale according to the invention can be favourably performed in a system according to Figure 2, having three reaction systems (a), (b) and (c) set in parallel according to a particular form of embodiment of the present invention.

A reaction system (a) comprises at least one reservoir 1a for the educt mixture or according to a further form of embodiment a reservoir filled with a ketone and a reservoir filled with a diethylaminosulfur trifluoride as such or as a solution in an organic solvent which are mixed continuously to form the educt mixture.

According to figure 2, the reaction system (a) further comprises the reaction chamber 3a which is continuously fed with the educt mixture.

The flow of the educt mixture is regulated by a pump 2a to the reaction chamber 3a.

Before entering the reaction chambers 3a the educt mixture is advantageously filtered by a filter 5a, for example a sintered stainless steel filter with pores of 5 to 50 µm.

The reaction chambers 3a-c are advantageously made of a stainless steel tube of about 18 m with a volume of about 0.5

The reaction chamber 3a is kept at a temperature of about 90°C by an oil bath 7a with the temperature monitored and regulated by a thermometer 4a.

The product mixture in exit of the reaction chamber 3a is monitored by an infrared detector 15 and is finally quenched by an aqueous alkali solution contained in the reservoir 17, for example a KHCO₃ or a NaOH solution, in a cooled reservoir vessel 12 a-b.

The reaction system according to the invention can further comprise an extra toluol reservoir 13 and is preferably kept under nitrogen 16 at a pressure between 0.05 to 10 bar, preferably of 0.2 bar, above ambient pressure.

According to a particular form of embodiment of the present invention at least one valve 18a can be inserted in the reaction system to control the flow and the pressure in the reaction system.

The following is an example of a deoxofluorination of a ketone for the production of geminal difluoride according to the invention:

53.1 Kg of Estrone acetate, 85.5 Kg of diethylaminosulfur trifluoride (DAST, 3.1 mol-equivalents) are dissolved in 60.1 Kg of toluene at room temperature forming the educt mixture. The reservoir containing the educt mixture is connected to a reactor system. The educt mixture is filtered and then fed at 0.9 litre/hour in three reactor chambers (0.3 litre/hour/chamber) which are set in parallel at a temperature of 90 °C (Fig.2).

Each reactor chamber comprises an inside volume of 0.5 litre.

The system is kept working under a pressure of 0.2 bar above ambient pressure and under inert conditions.

Finally, the product mixture coming out from the reaction chambers is steadily monitored and quenched with an aqueous solution of NaOH 20% w/w in a cooled container.

The obtained difluoride product is purified according procedures well known in art.

The method and the reaction system according to the invention allows an high productivity of a geminal difluoride derivative due to the round-the-clock operating possibilities and the numbering up of the reaction chambers.

The invention is described above with reference to a preferred embodiment. It is nevertheless clear that the invention is susceptible to numerous variations within the framework of technical equivalents.

## Claims

1. A method for the deoxofluorination of a ketone for the production of a geminal difluoride compound in an industrial scale comprising the following steps:
- preparing an educt mixture comprising a ketone and a dialkylaminosulfur trifluoride,
- letting flow the educt mixture into a reaction chamber of defined length, diameter and volume, with a flow path having a width perpendicular to the direction of the flow in the range of 1-10 mm, at a temperature of at least 75 °C up to 120°C, preferably between 80 and 100 °C, to perform the deoxofluorination reaction and obtaining a product mixture
- quenching the product mixture with a basic aqueous solution in a cooled reservoir vessel.

2. A method according to claim 1
**characterized in that**
the ketone and the dialkylaminosulfur trifluoride of the educt mixture are mixed continuously directly before entering the reaction chamber.

3. A method according to claim 1 or 2
**characterized in that**
the educt mixture further comprises an organic solvent, preferably toluene.

4. A method according to any one of the claims 1 to 3
**characterized in that**
the educt mixture is filtered before entering in the reaction chamber.

5. A method according to any one of the claims 1 to 4
**characterized in that**
the educt mixture is treated under pressure between 0.05 to 10 bar above ambient pressure, preferably under a pressure between 0.1 to 0.5 bar above ambient pressure.

6. A method according to any one of the claims 1 to 5
**characterized in that**
the length of the reaction chamber is comprised between 1 and 30 meters and the diameter between 1 to 10 mm.

7. A method according to any one of the claims 1 to 6
**characterized in that**
the total volume of the reaction chamber is between 0.1 to 10 litres, preferably between 0.2 to 2 litres.

8. A method according to any one of the claims 1 to7
**characterized in that**
the flow rate of the educt mixture within the reaction chamber is between 0.1 and 10 litres per hour, preferably between 0.2 to 1 litre per hour.

9. A method according to any one of the claims 1 to 8
**characterized in that**
the residence time of the educt mixture in the reaction chamber is between 0.1 and 10 hours, preferably between 0.2 and 2 hours.

10. A method according to any one of the claims 1 to 9
**characterized in that**
the dialkylaminosulfur trifluoride is diethylaminosulfur trifluoride or bis(2-methoxyethyl)-aminosulfur trifluoride.

11. A method according to any one of the claims 1 to 10
**characterized in that**
the ketone is estrone or an ester thereof, particularly estrone acetate.

12. A method according to any one of the claims 1 to 11
**characterized in that**
the product mixture is quenched with the a KHCO₃ or NaOH aqueous solution.

13. A reaction system for the production of a geminal difluoride compound in an industrial scale comprising:
at least one reservoir filled with a ketone or a solution therof and at least one reservoir filled with a dialkylaminosulfur trifluoride or a solution therof, or at least reservoir for an educt mixture comprising a ketone and a dialkylaminosulfur trifluoride,
one or more reaction chambers of defined length, diameter and volume with a flow path having a width perpendicular to the direction of the flow in the range of 1-10 mm,
an heating system for the reaction chamber and at least one collector vessel for the reaction mixture in exit from the reaction chamber.

14. A reaction system according to claim 13
**characterized in that**
it comprises a filter before the at least one reaction chamber.

15. A reaction system according to claim 13 or 14
**characterized in that**
it comprises a pump before the reaction chamber.

16. A reaction system according to any one of the claims 13 to 15
**characterized in that**
the length of the reaction chamber is comprised between 1 and 30 meters and the diameter between 1 to 10 mm.

17. A reaction system according to any one of the claims 13 to 16
**characterized in that**
the total volume of the reaction chamber is between 0.1 to 10 litres, preferably between 0.2 to 2 litres.

18. A reaction system according to any one of the claims 12 to 17
**characterized in that**
the dialkylaminosulfur trifluoride is diethylaminosulfur trifluoride or bis(2-methoxyethyl)-aminosulfur trifluoride.

19. A reaction system according to any one of the claims 12 to 18
**characterized in that**
the ketone is estrone or an ester thereof, particularly estrone acetate.

20. Use of a reaction system according to claims according to any one of the claims 13 to 19
for the deoxofluorination of a ketone for the production of a geminal difluoride product in an industrial scale by means of a dialkylaminosulfur trifluoride.
